# EUROPEAN PATENT APPLICATION

(11) **EP 3 730 187 A1**
(43) Date of publication of application: **28.10.2020**
(21) Application number: 19171215.7
(22) Date of filing: 25.04.2019
(51) Int. Cl.: A61N 5/06, A61N 5/10

(54) **SINGLE-USE PATCH**

(71) Applicant: DoseVue NV, 3590 Diepenbeek (BE)
(72) Inventor: D'Agostino, Emiliano, 3590 Diepenbeek (BE); Plesu, Adrian, 3590 Diepenbeek (BE)
(74) Representative: Brantsandpatents bvba

(57) **Abstract**

The current invention concerns a single-use patch for appliance on a skin of a subject, comprising at least two and preferably three hollow sheaths suitable for temporarily housing a fiber optic, wherein said sheaths comprise two open ends, wherein said sheaths are positioned to form a therapeutic surface within said patch by providing a fixed end of the fiber optic ends; wherein said patch is preferably combined with a topical cover layer and an adhesive supporting layer suitable to be attached to the skin of a subject.

## Description

### TECHNICAL FIELD

The present invention relates to a single-use patch and, in particular a single-use patch for appliance on a skin of a subject suitable for radiotherapy.

### BACKGROUND

Radiation oncology is the medical specialty concerned with prescribing and delivering radiation, and is distinct from radiology - the use of radiation in medical imaging and diagnosis. Radiation may be prescribed by a radiation oncologist with intent to cure ("curative") or for adjuvant therapy. It may also be used as palliative treatment (where cure is not possible and the aim is for local disease control or symptomatic relief) or as therapeutic treatment (where the therapy has survival benefit and it can be curative). It is also common to combine radiation therapy with surgery, chemotherapy, hormone therapy, immunotherapy, or some combination of the four.

Most common cancer types can be treated with radiation therapy in some way. The precise treatment intent (curative, adjuvant, neoadjuvant, therapeutic, or palliative) will depend on the tumor type, location, and stage, as well as the general health of the patient. Total body irradiation (TBI) is a radiation therapy technique used to prepare the body to receive a bone marrow transplant. Brachytherapy, in which a radiation source is placed inside or next to the area requiring treatment, is another form of radiation therapy that minimizes exposure to healthy tissue during procedures to treat cancers of the breast, prostate and other organs.

There are many different types of radiation therapies. External beam radiation therapy (XRT) is delivered via two- or three-dimensional beams using linear accelerator machines and is commonly used to treat prostate, breast and other tumors. In XRT treatment of the prostate, as an example, radiation is directed along different axes to the target prostate, which is near the rectal wall and surrounds the urethra. Where the beams cross, the radiation dose is the highest, and thus the prostate can be preferentially targeted. Misdirected radiation beams may perforate the rectal wall causing radiation proctitis (rectal bleeding), as well as erectile dysfunction (ED), incontinence and other complications. In fact, as many as half of the treated men suffer from ED and/or incontinence. Thus, it can be seen that that narrowly targeting the radiation is critical for reducing side effects.

Brachytherapy (internal radiation therapy) is delivered by placing radiation source(s) inside or next to the area requiring treatment. Brachytherapy is commonly used as an effective treatment for cervical, prostate, breast, and skin cancer and can also be used to treat tumours in many other body sites.

Dosage is always an important concern in treating any tumor or disease using radiation therapy. The dose should be enough to kill malignant cells, but tightly targeted so as to minimize damage to the surrounding healthy tissue. However, since patient tissues and organs are rarely immobile, the oncologist must allow a slightly increased area target to allow for movements caused by e.g., breathing, peristalsis, muscle contractions, and the like, and still ensure the tumor or other diseased area is adequately treated. This additional treatment zone surrounding the target is known as a "margin".

Because of concern over dosimetry and dosage uniformity, many companies are developing dosimeters that allow real time radiation dosage measurements, so that dosage can be more precisely controlled, rather than estimated. In the past, implantable devices for oncology applications have been proposed to evaluate the radiation dose amount received in vivo at the tumor site. See, e.g., US 6402689, the contents of which are hereby incorporated by reference herein. Measuring the radiation at the tumor site in vivo can provide improved estimates of doses received. However, for certain tumor types or situations, including certain fluoroscopic procedures, one or more skin-mounted or external surface radiation dosimeters may be desirable and sufficient for clinical purposes.

US 2005090738 discloses one single-use dosimeter sensor patch onto the skin of the patient, wherein the patch is self-contained. Though the wireless patch is suitable to apply at any desired skin part of a subject, it does not offer a real-time monitoring.

WO 2016075717 discloses an ionizing radiation detector comprising a plurality of scintillators of plastic material. It however meets the problem of adapting to irregular surface of a skin.

Current gap for designing a single-use patch suitable for radiotherapy is that it should i) be suitable for temporarily housing fiber optics; ii) specify a fiducial marker for target area; iii) not affecting the radiation dose; iv) be moldable to irregular surface of a skin; v) adaptable to different working diameter and shape; vi) be at ease for storage and transportation.

### SUMMARY OF THE INVENTION

In a first aspect, the invention provides a single-use patch for appliance on a skin of a subject, comprising at least two and preferably three hollow sheaths suitable for temporarily housing a fiber optic, wherein said sheaths comprise two open ends, wherein said sheaths are positioned to form a therapeutic surface within said patch by providing a fixed end of the fiber optic ends; wherein said patch is preferably combined with a topical cover layer and an adhesive supporting layer suitable to be attached to the skin of a subject.

The inventors have thus realized the a fiber optic housed by a hollow sheath measure the dose of radiation reaches at the end tip of a fiber optic. With comparison between the detected dose at the end tip of each fiber optic, it further allows to detect whether radiation reach the skin of a subject uniformly.

In a second aspect, the present invention provides a kit of single-use radiation dosimeter patches, in a subject in need thereof, the kit comprising:
- single-use patches with different working diameters;
- a plurality of said patches in each working parameters.

### DESCRIPTION OF FIGURES

**Fig. 1** shows schematically a perspective view of the patch in a preferred embodiment.
**Fig. 2** shows schematically a perspective view of the patch when all components are disassembled.
**Fig. 3A** shows schematically a front view of the patch, where a fiducial circular marker is drawn on the topical cover layer.
**Fig. 4** shows schematically a perspective view of the cross-section of the patch.
**Fig. 5** shows schematically a perspective view of the patch in an alternative embodiment.
**Fig. 6** shows schematically a perspective view of when the patch is applied to a subject.
**Fig. 7A** shows schematically a perspective view of the invention in a further preferred embodiment - a shield, wherein the components are disassembled. **Fig. 7B** shows schematically a perspective view of said shield, wherein components are assembled.
**Fig. 8A** shows schematically a perspective view of the invention in a further preferred embodiment - a probe. **Fig. 8B** shows schematically a perspective view of the cross-section of said probe. **Fig. 8C** shows schematically a perspective view of the probe when ready for appliance.

### DETAILED DESCRIPTION OF THE INVENTION

The present invention concerns positioning element for single-use patch.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

### A single-use patch

In a first aspect, the invention provides a single-use patch for appliance on a skin of a subject, comprising at least two and preferably three hollow sheaths suitable for temporarily housing a fiber optic, wherein said sheaths comprise two open ends, wherein said sheaths are positioned to form a therapeutic surface within said patch by providing a fixed end of the fiber optic ends; wherein said patch is preferably combined with a topical cover layer and an adhesive supporting layer suitable to be attached to the skin of a subject.

The inventors have thus realized the a fiber optic housed by a hollow sheath measure the dose of radiation reaches at the end tip of a fiber optic. With comparison between the detected dose at the end tip of each fiber optic, it further allows to detect whether radiation reach the skin of a subject uniformly.

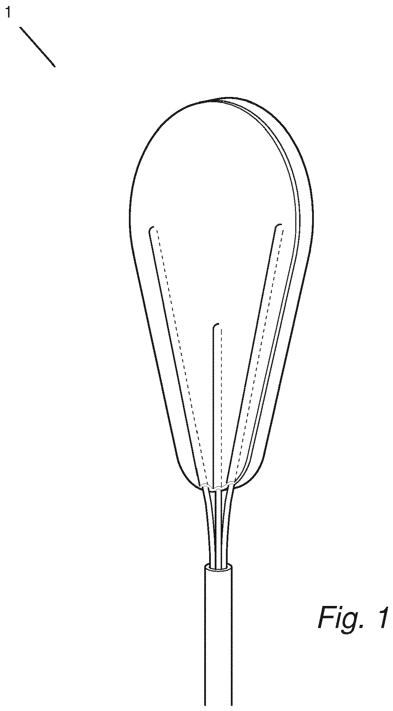

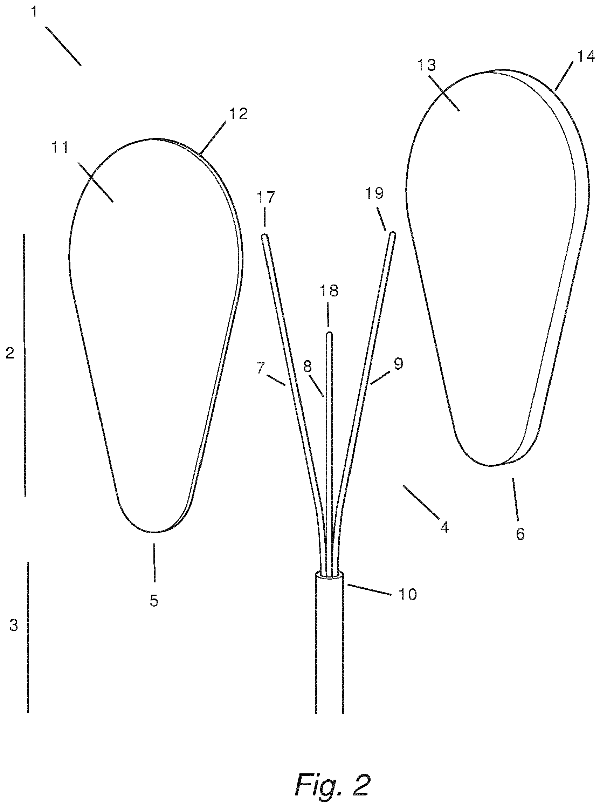

In a preferred embodiment (Fig. 1 and 2), the present invention provides a patch (2) according to the first aspect of the invention, comprising three hollow sheaths (4), that are suitable for temporarily housing three fiber optics (7, 8, 9). The hollow sheaths are typically made in for instance, but not restrict to peek or PTFE or Vestamid Care polymer, that is reliable for housing a fiber optic and moldable to irregular surface at the same time. The end tips of each corresponding fiber optic are inserted up to the end of hollow sheaths (17, 18, 19). On the other end, three fiber optics (7, 8, 9) are gathered in a tube (10) where eventually will connect the fiber optics to a monitoring machine for radiation dose data analysis.

In a preferred embodiment, the patch (2) further comprises a cover layer (5), wherein one side (12) is covering the hollow sheaths and the other side comprises a fiducial circular marker (15). The end tips (17, 18, 19) of the hollow sheaths form a triangle surface and lie on the circumference of said fiducial marker (15). The area (16) of said fiducial marker (15) is the therapeutic surface and is expected to fully cover the targeting area for the treatment. The size of this area (16) is referred as "working diameter" in this document.

In a preferred embodiment, the patch (2) further comprises an adhesive layer (6), wherein on one side (13), it functions as a supporting layer of the hollow sheaths (4); while on the other side (14), it directly touches the skin of a subject, which requires it to be moldable to irregular surface.

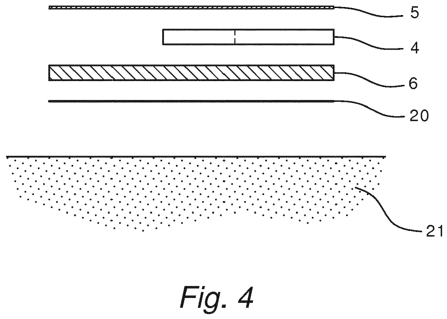

In a preferred embodiment, the patch (2) further comprises a peel-off layer (20), as shown in Fig. 4, which allows the patch (2) stored and transported in a sterile environment.

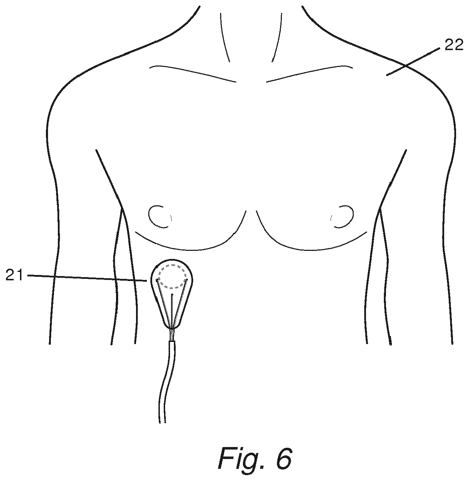

During a irradiation (Fig. 6), a patch (2) is first assembled with fiber optics (7, 8, 9). The assembled patch (1) is then applied on the skin of the targeting area (21) of a subject (22). The irradiation is first passing through the cover layer (5), followed by the end tips of fiber optics fixed at the ends (17, 18, 19) of hallow sheaths (4), and the adhesive layer (6), before reaching the target (e.g. a tumor). Note that i) the thickness of said patch (2) shall not affect the irradiation; ii) the fiber optics (7, 8, 9) shall not affect the dose that delivered.

During a irradiation (Fig. 6), the irradiation is passing through the assembled patch (1), more specifically the working area (16). The end tips of fiber optics (7, 8, 9) are measuring the dose passing through without interfering with the dose delivered. In an ideal situation, the working area (16) is expected to fully cover the targeting area (e.g. a tumor), but be restricted to a certain size at the same time. Thus, the challenge is about the correct working diameter and the precise location on a skin of a subject. Furthermore, during the radiotherapy, the patch (2) needs to stay fixed for a stable real-time dose monitoring, which is up to the attachment of adhesive layer (6) on a skin. The cable part (3) which is eventually linked to the monitoring machine, needs to be flexible, as the patch might be applied on any possible part of a subject (22).

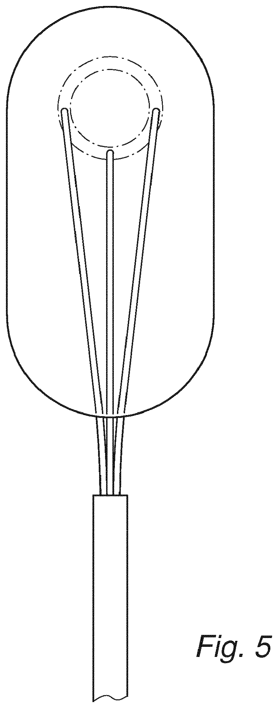

Though this might be critical about the working diameter, i.e. fiducial circular marker (15) and the working area (16). However, the shape of the patch is never restricted to what is shown in current preferred embodiment. Fig. 5 shows an alternative shape of the patch. Noted that the shape of the patch could be adjusted to any form suitable for the use. Same principle also applies to the working area (16).

### A shield

Though the eternal goal of the dose monitoring in radiotherapy is to minimize it that it is precisely enough for the treatment purpose. It is foreseen that there is always remaining dose in practise. To date, it is often seen that a shield or a stopper is positioned behind the target (e.g. a tumor) in between tissues, as it will absorb the remaining radiation and thus protects other organs. However, it is rarely concerned about the exact dose that a shield absorbes.

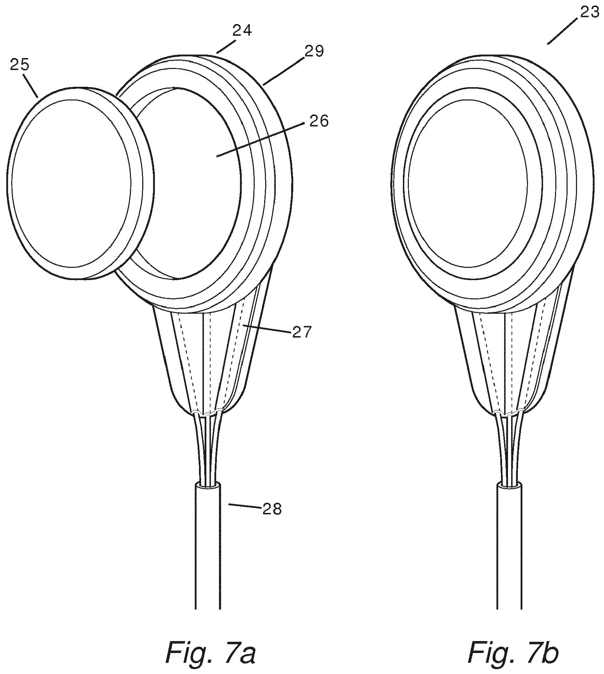

In a further preferred embodiment (Fig. 7A), the present invention provides a shield (23), comprising a housing (24), hollow sheaths (27) suitable for housing fiber optics, an absorber (25), and filler (26).

Similar to the previous embodiment (Fig. 1-3), on one surface (29) of the shield (23), a fiducial circular marker is drawn, and the end tips of hollow sheaths (27) lie on the marker, with same functions and purposes as disclosed in the previous embodiment. When in use, the end tips of the fiber optics are inserted to the end of hollows sheaths (27), and the other end of the fiber optics are gathered in a tube (28) which eventually connecting the fiber optics to a monitoring machine for radiation dose data analysis.

An absorber (25) is shown apart in Fig. 7A. Correspondingly, the absorber (25) is positioned after the hollow sheaths (27), and fully covers the working area marked on the surface (29). When assembled (Fig. 7B), the hollow sheaths (27) stands in the middle between the absorber (28) and the surface (29) of the housing (24). It is essential that the components shall be relatively stable. The filler (26), typically a silicone grade silicon rubber, for instance, is thus to fix the positions of all components.

During an irradiation, three fiber optics are first inserted in the hollow sheaths (27), the shield (23) is then positioned at the opposite side of the target (e.g. a tumor) to the radiation source. More specifically, the surface (29) of the housing (24), where a fiducial circular marker is drawn, is proximal to the target (e.g. a tumor). Whereas the absorber is distal to the target (e.g. a tumor).

It has to be noted that when assembled in operation, the absorber (25) is positioned in the housing (24) with a single-use tape. Thus the absorber (25) is recyclable after the treatment, while other components within said shield (23) are single-use. The recycling process of said absorber (25) however needs to be handled by professions and related authorities.

Said shield is real-time monitoring the remaining dose after the irradiation to the target. The advantage of said shield, if combined with the previous embodiment - patch, could contribute to i) diagnose the irradiation dose applied to the subject; ii) build mathematical model of the target, even possibly in 3D if dose data is sufficient; iii) real-time monitor the attenuation process of the target during the irradiation; iv) build database for the treatment of current target, benefiting the dosage appliance in future similar therapies.

Assuredly, to achieve the said advantages, a series of challenges need to be carefully accomplished, such as the position synchronization of end tips of fiber optics in said patch and in said shield. The said advantages give a limited view of potential application possibilities. Certainly, it shall not restrict the scope of current invention.

### A single-use probe

There are circumstance that an in-vivo dosimeter is needed when the target is not close to the skin. The diameters of the dosimeter is thus restricted to an acceptable level.

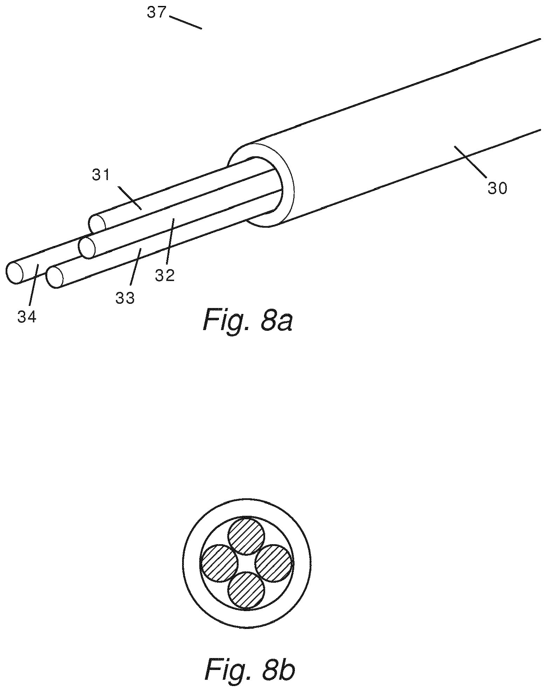

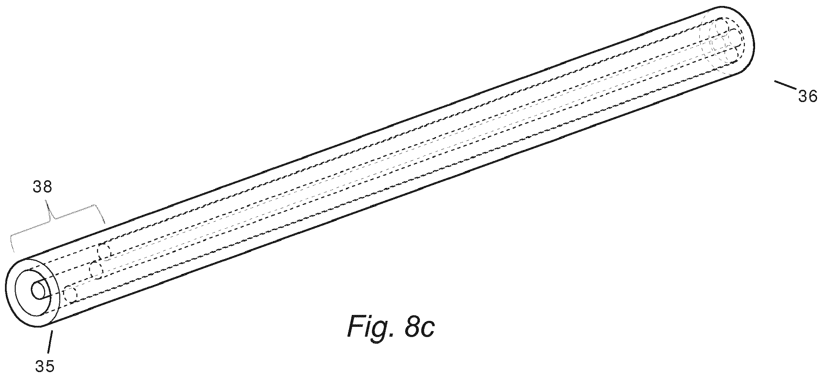

In a further preferred embodiment (Fig. 8A), the present invention provides a probe, comprising a housing tube (30), four hollow sheathes (31, 32, 33, 34) suitable for temporarily housing four fiber optics. Said hollow sheaths (31, 32, 33, 34) come in an ascending or descending length at the proximal end (35). The housing tube (30) shall fully cover the hollow sheaths axially, as shown in Fig. 8C.

When in use, fiber optics are first inserted to the end of said hollow sheaths (31, 32, 33, 34), the proximal end (35) is then positioned at the desired location. The end tips of fiber optics measures the dose passing through the working area (38). The distal end (36) is eventually linked to a monitoring machine that receives the single captured by the end tips of fiber optics.

The advantage of said probe mainly concerns the flexibility of appliance due to its size. It is greatly desirable for situations that the target treating area is hard to reach. Moreover, for irregular shape of lesion (e.g. fusiform), it is more desired to monitor the radiation dose axially. Note that current embodiment does not restrict the number of hallow sheaths (correspondingly the fiber optics), and the working area (38). They shall be adjusted based on the exact event for the best benefit of the treatment.

### A sterile kit of single-use patches

In a second aspect, the present invention provides a kit of single-use radiation dosimeter patches, in a subject in need thereof, the kit comprising:
- single-use patches with different working diameters;
- a plurality of said patches in each working parameters.

In a preferred embodiment, said kit comprises single-use patches in different working diameter and different forms such as shown in previous disclosed embodiments. Variance could apply to the number of hollow sheaths, the series of working diameters, the suitable shapes or forms and corresponding size. Said kit shall offer a sterile environment for the devices stored inside.

Preferably, said kit is configured to offer devices according to the first aspect of the invention. The possible devices (patch, shield, probe, etc.) in the kit shall come along with a mature supply and logistics.

Using the preferred embodiments disclosed in this document to summarize the advantages of current invention in first aspect - the patch and in second aspect - the kit, it is that, in a radiotherapy, i) it offers an easy assembling for a surgeon/nurse to insert fiber optics; ii) it is suitable for irregular surface of a skin; iii) it comes in different working diameters, shapes and forms, which a surgeon/nurse could choose a most suitable one; iv) it is highly replaceable with a mature supply chain and logistics.

It has to be noted that the preferred embodiment nevertheless is used to disclosed the present invention, it however, never restricts the scope of the invention, including but not restrict to: the forms and shapes of the patch, the using situations and combinations, etc.

### EXAMPLE

A preferred embodiment of the present invention is presented below. It is however clear that other embodiments can easily be contemplated within the scope of the invention. The presentation below should thus not be construed restrictive, but it should be realized that the skilled person will easily apply modifications to the presented example without reappraisal of the appended claims.

### EXAMPLE 1

A preferred embodiment is shown via the example when applying on a skin cancer treatment.

In the treatment of skin cancer, it is important to be able to monitor the dose delivered in the proximity of the lesion and to monitor, at the same time, that a uniform radiation field is reaching the tumoral lesion. To this end, before starting the treatment, the dermatologist applies an adhesive patch on the lesion. It has to be noted that the patch is extremely thin and does not as such interfere with the dose delivered to the lesion. In the patch there are 3 hollow sheaths that reach the edges of a circle (fiducial marker) entirely covering the lesion. The fiducial marker is drawn on the topical layer of the patch. The end tips of these sheaths are at 120° from each other evenly distributed on the edge of fiducial marker. In each sheath, one radiation fiber sensor is inserted before starting the treatment. When irradiating, the same value is expected at each of the 3 fiber sensors, if not, a lack of uniformity in the radiation field is signaled. When the radiation detectors reach the total doses needed to sterilize the lesion, a visual/acoustic signal (e.g. a tone) is given to the operator so that the irradiation machine can be stopped.

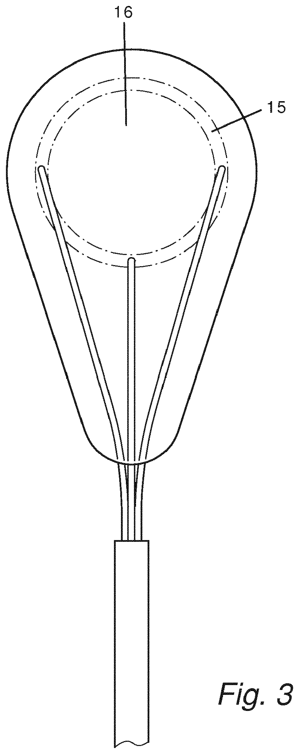

Fig. 3 shows one of the possibilities for above said patch in terms of shape and size. Alternatives, such as the shape shown in Fig. 5 are nevertheless also possible for the said patch. The working diameter of said fiducial marker shall be adjusted according to the requirement of the treatment.

### EXAMPLE 2

An alternative embodiment of the invention is a shield for absorbing the remaining radiation does after the irradiation treatment.

There are three general guidelines for controlling exposure to ionizing radiation: minimizing exposure time, maximizing distance from the radiation source, shielding from the radiation source. As ionizing radiation passes through matter, the intensity of the radiation is diminished. Though nowadays technology attempts to give precise dose of irradiation to the tumor, which ideally will be totally diminished. However, it is almost never the case in a radiotherapy. In practice, to ensure enough dose is applied to the tumor from all directions, the treatment often gives more irradiation dose. As such the direct result is the remaining dose will keep irradiating other organs behind the tumor unnecessarily, where a shield is exactly needed to prevent it. A shield is the placement of an "absorber" between other organs and the radiation source, using as an absorber that reduces remaining radiation. Alpha, beta, or gamma radiation can all be stopped by different thicknesses of absorbers.

To date, it is often seen that a shield is positioned behind the tumor in between tissues in radiotherapy, for the above-said matter. However, it is rarely concerned that the dose absorbed by the shield. Such information could help to diagnose the irradiation dose applied to the tumor, especially combined with the dose information monitored by the embodiment shown in Example 1. It can further contribute to the mathematic modelling of the tumor if the monitoring data is sufficient. The comparison between the obtained modelling of the tumor and previous scanned result before the radiotherapy could be applied, which eventually could contribute to the calibration of the modelling of a tumor. Moreover, with sufficient input, such these process could potentially contribute to build a database that might plan the optimized precise dose for a tumor before the irradiation treatment. In all cases, the goal is to minimize the dose that will be absorbed by the shield, without affecting sufficient radiation to the tumor.

The embodied device according to Example 2 and Fig. 7 corresponds to the embodiment according to Example 1 and Fig. 1,

### EXAMPLE 3

A further embodiment of the invention is a probe for brachytherapy.

During brachytherapy treatment of cervix cancer, an applicator is inserted in the vagina of the patient. Within this applicator channels are present where a radioactive source, fixed on the tip of a cable, is temporarily brought in the patient. As of today, there is no way to verify that the source is currently brought into each of the channels, as planned. The position of the source in the channels will eventually determine the entire 3D dose distribution in the patient.

In order to be able to verify, in real-time, the current position of the source, the radiation therapist fasten a probe on the applicator. As shown in Fig. xx, this probe comprises a tube having 4 channels wherein optical fiber sensors are inserted. The four channels decrease progressively in length, and the end tips are positioned evenly at the inserted end. By combining the distance-dependent signal of each of the 4 sensors, a dedicated software module, in capable of determining the position of the radioactive source in the applicator. In case the detected source position would differ significantly (e.g. more than 1mm) from the expected position, than an alarm signal is sent to the operator, who can decide to stop the treatment.

The embodied device according to Example 3 and Fig. 8 corresponds to the embodiment according to Example 1 and Fig. 1.

## Claims

1. A single-use patch for appliance on a skin of a subject, comprising at least two and preferably three hollow sheaths suitable for temporarily housing a fiber optic, wherein said sheaths comprise two open ends, wherein said sheaths are positioned to form a therapeutic surface within said patch by providing a fixed end of the fiber optic ends; wherein said patch is preferably combined with a topical cover layer and an adhesive supporting layer suitable to be attached to the skin of a subject.

2. A single-use patch of claim 1, comprising three hollow sheaths, wherein the end tips of said three sheaths form a triangular therapeutic surface.

3. A single-use patch of claim 1, further comprising a topical cover layer and an adhesive supporting layer.

4. A single-use patch of claim 1 to 3, further comprising a fiducial circular marker drawn on the topical cover layer for alignment of said patch to a target treatment area.

5. A single-use patch of claim 1 to 4, wherein said end tips of said three hollow sheaths lie on the circumference of said fiducial marker.

6. A single-use patch of claim 1 to 5, wherein said triangular therapeutic surface is fully covering the targeting area for the treatment.

7. A positioning element of claim 1, wherein the diameter of said fiber optic is not bigger than said hollow sheaths.

8. A patch of claim 1 and 3, wherein the end tips of fiber optics are measuring the radiation dose without interfering with the dose delivered.

9. A positioning element of claim 1, wherein said hollow sheaths are of a biocompatible polymer such as PTFE or peek.

10. A single-use patch of claim 1 and 3, wherein said topical layer is moldable to irregular surface.

11. A single-use patch of claim 1 and 3, wherein said adhesive layer is moldable to irregular surface.

12. A single-use patch of claim 1 and 3, further comprising a peel-off layer.

13. A single-use patch of claim 1 to 11, wherein the thickness of the patch is not more than 1 mm.

14. A single-use patch according to claims 1 to 13 is of use in a radiation dosimeter in radiotherapy.

15. A sterile medical kit of single-use radiation dosimeter patches of claim 1 to 16, the kit comprising: single-use patches with different working diameters, a plurality of said patches in each working parameters.
